# EUROPEAN PATENT SPECIFICATION

(11) **EP 4 089 179 B1**
(45) Date of publication and mention of the grant of the patent: **31.07.2024**
(21) Application number: 21207989.1
(22) Date of filing: 12.11.2021
(51) Int. Cl.: C12Q 1/04, C12Q 1/18

(54) **PROCESSES FOR MICROBIOLOGICAL DETECTION AND DETERMINATION OF ANTIMICROBIAL SUSCEPTIBILITY IN CLINICAL, ENVIRONMENTAL OR FOOD SAMPLES**
VERFAHREN ZUR MIKROBIOLOGISCHEN DETEKTION UND BESTIMMUNG DER ANTIMIKROBIELLEN EMPFINDLICHKEIT IN KLINISCHEN, UMWELT- ODER LEBENSMITTELPROBEN
PROCÉDÉS DE DÉTECTION MICROBIOLOGIQUE ET DE DÉTERMINATION DE LA SENSIBILITÉ ANTIMICROBIENNE DANS DES ÉCHANTILLONS CLINIQUES, ENVIRONNEMENTAUX OU ALIMENTAIRES

(30) Priority: 12.05.2021 PT 2021117223
(43) Date of publication of application: 16.11.2022
(73) Proprietor: Instituto Politécnico De Leiria, 2411-901 Leiria (PT)
(72) Inventor: DA FONSECA PINTO, Rui Manuel, 2411-901 LEIRIA (PT); DOS SANTOS GONÇALVES PEREIRA, Sónia Margarida, 2411-901 LEIRIA (PT)
(74) Representative: Monteiro Alves, Inês

(56) References cited:
- WO-A1-2017/218202
- US-A1- 2012 149 599
- US-A1- 2013 217 063
- US-A1- 2018 216 155
- LOUTFI HADI ET AL: "Real-time monitoring of bacterial growth kinetics in suspensions using laser speckle imaging", SCIENTIFIC REPORTS, vol. 10, no. 1, 1 December 2020 (2020-12-01), pages 408, XP055910220, Retrieved from the Internet <URL:https://www.nature.com/articles/s41598-019-57281-2.pdf> DOI: 10.1038/s41598-019-57281-2
- SEUNGYUN HANHOJUN NOYOONSEOK BAEKHUIJUN PARKKYEOREH LEESEUNGBUM YANGYONGKEUN PARK: "Rapid antimicrobial susceptibility test using spatiotemporal analysis of laser speckle dynamics of bacterial colonies", BIORXIV, 25 November 2019 (2019-11-25), XP002806196, Retrieved from the Internet <URL:https://www.biorxiv.org/content/10.1101/853168v1> DOI: 10.1101/853168

## Description

### Field of application

The present invention refers to a process for the detection of any type of microorganism, including bacteria, fungi and viruses, in clinical, environmental or food samples, having as a basis optical interference properties by light reflection.

The process of the present invention also allows to obtain a microbiological profile for the testing of antimicrobials, when the microbiological detection indicates the presence of bacteria or fungi, by the controlled release of pre-defined and progressively increasing amounts of antimicrobial to the sample in a matrix of reservoirs.

### State of the art

There currently exists an ever increasing urgent need to identify microorganisms such as bacteria, fungi and viruses (encapsulated or not), which can be present in clinical, environmental or food samples, to enable the use, for bacteria and fungi, of the adequate antimicrobial or, in the case of viruses, that the same be simply detected. This quick microbial identification will allow accelerating the treatment and provide a more rapid recovery of the host or the environment wherein these pathogens are present.

There have been several attempts to make this identification in a rapid manner. However, up to the present moment, hours and even days have been necessary for this identification and determination of antimicrobial susceptibility to occur, leading to the pathogen remaining without identification and continuing to trouble the sample, host or environment in which it is located, since its antimicrobial susceptibility is still unknown.

Document WO2017198240A1 refers to an optical stimulation system (optical photostimulation) for microorganisms in biological samples which uses a stimulation signal generator, LED light emitters and other devices including software, which detect the presence of these microorganisms. It is a complex system which, apart from the above, further requires the previous cultivation of the culture to be analyzed, thus taking at least 12h (see example 1 of said document), for the sample to be analyzed and the microorganisms identified by the method proposed in this document.

Document JP2019088339A aims at providing a technique for the rapid bacterial identification and determine a sensitivity test for a medication. In this document, the situation of the bacterial division is monitored observing the shape and number of each well of culture plate for bacterial identification and by performing a a drug sensitivity test by microscope.

Thus, the shape, number and area of the bacteria are analyzed from the images obtained by the microscopic observation to see whether the bacterial growth is in the induction period into a logarithmic period, and the changes over time are graphically represented. From the graph, it is determined whether there exists or not bacterial growth in each measurement, and the result of the drug sensitivity is exhibited on the screen to provide the drug sensitivity result for each measurement. In this document a culture medium is equally used leading to a considerable increase in the analysis time.

Document (https://www.biorxiv.org/content/10.1101/853168v1.full - SeungYun Han, Hojun No, YoonSeok Baek, Huijun Park, KyeoReh Lee, Seungbum Yang, YongKeun Park - Rapid antimicrobial susceptibility test using spatiotemporal analysis of laser speckle dynamics of bacterial colonies, November 25, 2019) refers to a bacterial identification process and to a susceptibility test for a drug in a rapid manner. It is a rapid test in the detection stage. However, it still resorts to the culture of the species presented in the sample, and for which there already is identification. The presence of this step in the process described in the referred document will result in the analysis not being as rapid as desired. In fact, the use of the culture step provides an analysis which will take from 12 to 24h.

Therefore, it is extremely important for the rapid recovery of a patient, the prevention of the propagation of a hospital infection and/or the suppression of the appearance of a bacteria that is resistant to a drug or even the prevention of the propagation of a virus, that there exist a process which provides the execution of a rapid pathogen identification test and the subsequent drug sensitivity test for an antimicrobial, bactericide or fungicide, if applicable, for the treatment of this same pathogen to be carried out in a rapid and assertive manner. In the case of a virus, the identification of the presence thereof and the possibility of the subsequent genetic sequencing, in case this is of interest, will allow acting over the same in a quick manner, allowing prevention against the propagation thereof in the system.

The present invention solves, in a surprising manner, this question, that is, the processes of the present invention allow detecting the presence of microorganisms, whether they are bacteria, fungi or viruses, in a clinical, environmental and food sample in a rapid manner. Even more important than identifying the microorganisms, the present invention is capable of recognizing the antimicrobial susceptibility profile so one can rapidly and effectively act.

Rapid microorganism identification systems already exist and are several, however, the present invention allows to accelerate the component of the antimicrobial susceptibility profile, which is still very time consuming.

The processes of the present invention include a sample concentration step, in order to concentrate the microbiological component contained in that same sample, eliminating the "noise" that it contains, only maintaining present the microorganisms in the same. The eliminated "noise" will vary from sample to sample, as it is known beforehand what should or not be removed from same. This concentration step allows the increase of the detection resolution. Apart from the concentration, it is also important the fact that the process of the present invention is centered on the coherent light characteristics and the reflection and/or refraction thereof allowing the detection and characterization of the micromovements of the microorganisms, in the case of bacteria and fungi, or the variation in cell line growth, in the case of viruses. In fact, the presence of these micromovements will confirm the existence of the microorganisms in the sample, more specifically these micromovements detect the bacterial or fungal growth. Subsequently to the confirmation of the presence of microorganisms in the sample, in the case of bacteria and fungi, the antimicrobial susceptibility test will be carried out to identify in an assertive manner which antimicrobial should be used in the treatment of the pathogen. The process of the present invention thus allows reducing the detection of microorganisms and antimicrobial test, from 12 to 24h - minimum time necessary for culture growth - which step does not exist in the process of the present invention - in face of the known processes, in this manner allowing the assertive empiric prescription of the antimicrobials, minimizing risks for the health of the patient and the public health.

Another advantage of the present invention resides in the fact that the samples to be analyzed can be of biological/clinical (human or veterinarian), food or environmental origin. In fact, the process of the present invention can be applied in clinical samples which can be submitted to microbiological analysis for research on pathogenic microorganisms and resistance to antimicrobials (i.e., blood, urine, feces, cerebrospinal fluid, biopsies, sputum, saliva, exudates, washes, scrapes, smears), in food samples (in the scope of food quality control and preservation of food, such as milk), and also in environmental samples (particularly water and soil).

It is thus possible, with the process of the present invention, to detect, in the case of bacteria and fungi, the antimicrobial susceptibility profiles of the prevalent pathogenic microorganisms and which are most worrying in terms of antibiotic therapy worldwide, such as, but not limited to, microorganisms having rapid growth, *Enterococcus faecium, Staphylococcus aureus, Klebsiella pneumoniae, Acinetobacter baumannii, Pseudomonas aeruginosa, Enterobacter sp., Escherichia coli,* and microorganisms having fastidious growth, *Legionella pneumophila, Mycobacterium tuberculosis* among others, as well as fungi. The process of the present invention further allows detecting the presence of the virus in the sample to be analyzed.

Therefore, it is extremely universal processes as to the type of microorganisms to be detected, as well as regarding the medium wherein the same can be located.

### Summary of the Invention

Are objects of the present invention the processes of microbiological detection (bacteria, fungi and viruses) and antimicrobial susceptibility determination, when justified, in clinical, environmental or food samples, in a rapid manner.

In a first aspect of the invention, the process of microbiological detection and antimicrobial susceptibility determination in clinical, environmental or food samples, comprises the following steps:
i) Concentration of the microbiological component of a sample to be analyzed;
ii) Incubation of the sample, obtained in step i), and addition of a volume of a concentrated liquid culture medium; wherein the ratio between the volume of the culture medium and the volume of the concentrated sample is of 1: 1 and is in the range of 200 µL to 2 mL;
iii) Introduction of the concentrated and enriched sample, obtained in step ii), in a reservoir matrix comprising at least two wells and at least two controlled release reservoirs of antimicrobial agents, wherein each well is configured to receive a portion of the sample and is associated to a controlled release reservoir of antimicrobial agents, wherein each controlled release reservoir of antimicrobial agents is configured to release an antimicrobial agent over a portion of the sample;
iv) First analysis of the sample enriched with the culture medium, obtained in step ii), by irradiating it with external energy and subsequent digital recording of the information from the first analysis to detect the presence of micromovements, wherein the external energy is originated from coherent light sources having wavelengths varying in the range from 193 nm to 2940 nm, preferably 690 to 1064 nm, and powers varying in a range from 0,1 mW to 400 mW, preferably from 0,5 mW to 100 mW;
v) Controlled release of a first amount of at least one antimicrobial agent, from a controlled release reservoir of antimicrobial agents, over a portion of the sample obtained in step ii), which is disposed in the well associated with the respective controlled release reservoir of antimicrobial agents;
vi) Second analysis of the sample obtained in step v), which is again irradiated with external energy and subsequent digital recording of information from the second analysis;
vii) Processing of the information obtained in steps iv) and vi) using a suitable processing algorithm, with the antimicrobial susceptibility profile being issued to the end user as a result of each sample from step v);

wherein steps v) and vi) are repeated at least once, wherein in a repetition of step v) the controlled release of a second amount of at least one antimicrobial agent from an controlled release reservoir of antimicrobial agents occurs, over the respective associated well including the sample portion and in which in a repetition of step vi) the digital recording of the corresponding analysis information is performed; and
wherein the microorganisms to be detected are selected from the group which consists of bacteria or fungi having rapid growth or fastidious growth.

In a second aspect of the invention, the process for viral detection in clinical, environmental or food samples comprises the following steps:
i) Concentration of the microbiological component of a sample to be analyzed;
ii) Transfer of the sample, obtained in step i), to a cell line; and
iii) Analysis of the sample obtained in step ii), which is irradiated by means of the external energy and subsequent execution of the digital recording of the information from the analysis, to detect the virus, wherein the external energy is originated from coherent light sources having wavelengths varying in the range from 193 nm to 2940 nm, preferably 690 to 1064 nm, and powers varying in a range from 0,1 mW to 400 mW, preferably from 0,5 mW to 100 mW; and
iv) Processing of the information obtained in step iii) using a suitable processing algorithm, with the result being issued to the final user in the form of detected / not detected binary data for each sample of step i);
wherein the microorganisms to be detected are viruses.

As can be verified, the invention refers to extremely useful and universal processes, adequate for the rapid microbiological detection in varied samples, both in routine laboratory activities as in occasions where a rapid form of detection is necessary for taking action.

### Detailed description of the Technology

The present invention refers to processes for the detection of any type of microorganisms, whether they are bacteria, fungi or viruses, and the determination of the antimicrobial susceptibility profile thereof (when justified, *i.e.,* in the case of bacteria and fungi), directly from clinical, environmental or food samples, based on optical interference properties by light reflection, to guide the assertive medical prescription of antimicrobials in real time, *i.e.,* in a clinically adequate time window, and which will enable quick action over the pathogen.

In one preferred embodiment, the process of the present invention can be applied to clinical samples which can be submitted to microbiological analysis for research on pathogenic microorganisms and, in the case of samples that comprise bacteria or fungi, also the resistance to antimicrobials (i.e., blood, urine, feces, cerebrospinal fluid, biopsies, sputum, saliva, exudates, washes, scrapes, smears), in food samples (in the scope of food quality control and preservation of food, such as milk), and also in environmental samples (particularly water and soil). It is thus possible, with the process of the present invention, to detect the antimicrobial susceptibility profiles of the prevalent pathogenic microorganisms and which are most worrying in terms of antibiotic therapy worldwide, such as, but not limited to, microorganisms having rapid growth, *Enterococcus faecium, Staphylococcus aureus, Klebsiella pneumoniae, Acinetobacter baumannii, Pseudomonas aeruginosa, Enterobacter sp., Escherichia coli,* and microorganisms having fastidious growth, *Legionella pneumophila, Mycobacterium tuberculosis* among others, as well as fungi.

The processes of the present invention focus on the characteristics of coherent light and its reflection and/or refraction that allows detecting and characterizing micromoviments, useful to detect the growth of microorganisms (bacterial or fungal) or the growth variation in the cell lines by viral action together with clinical sample concentration processes to increase detection resolution.

In a preferred embodiment, the external energy refers to sources of coherent light (such as laser), wherein, the elements and wavelengths are situated in the range that varies from 193 nm to 2940 nm, preferably 690 nm to 1064 nm. This range is adaptable to the dimension, morphology and type of movement of the microorganisms for the samples mentioned in the present patent application. In fact, this range of wavelength is made to run on the sample or samples being analyzed until the presence of microorganisms in the same is detected. Subsequently to this run it is possible to perceive which is the adequate wavelength for the sample under analysis.

In another preferred embodiment, the coherent light sources are chosen between argon fluoride (ultraviolet), the Helium-Neon (red or green), YAG or carbon dioxide. These light sources are stable light sources.

In another preferred embodiment the powers vary in a range from 0,1 to 100 milliwatts (mW), preferably from 0,5 mW to 100 mW, which will be adapted to the sample being analyzed. Such as referred in the case of the wavelengths, this range of powers is adaptable to the dimension, morphology and type of movement of the microorganisms.

The most general and advantageous configurations of the present invention are detailed below in conformity with other preferred and/or advantageous forms of embodiment of the present invention.

The object of the present invention is a microbiological detection process, in which the microorganisms to be detected are selected from the group consisting of fast-growing or fastidious bacteria or fungi, and determination of antimicrobial susceptibility in clinical, environmental and food samples comprising the following steps:
i) Concentration of the microbiological component of a sample to be analyzed

The concentration of the sample is carried out by means of several techniques that are known in the art, on their own or in progressive steps, depending on the type of sample, such as: adsorption (by ionic change in resin or metallic hydroxides), partition (in two-phase aqueous phase), centrifugation (simple, differential or density gradient), filtration (simple, electropositive or negative), selective immobilization, separation by biochemical affinity or immune magnetic (lectin, antibodies, bacteriophages or their ligands), among others, all in a macro or micro volumetric scale (liter to microliter). The technique to be used will depend on the sample to be purified, whereby the referred techniques are well known to a person skilled in the art. In one aspect of the invention, the final concentration will have volumes that vary between the milli or microliters, preferably, the volume of the concentrated sample is in the range of 200 µL to 2 mL.

In one preferred embodiment, upon conclusion of the concentration step, the temperature of the concentrated sample is stabilized in the range from 22 °C to 45 °C according to the type of sample to be analyzed.

ii) Incubation of the concentrated sample, obtained in step i), and addition of a volume of a concentrated liquid culture medium, which has the purpose of increasing the "detectability" of the microorganisms to guarantee an adequate concentration of signal for the limit of optical detection of the analysis process. This limit depends on the sample being analyzed.

In one preferred embodiment the enriched sample remains in incubation for a period which may be between minutes and hours, whereby this period is less or equal to 12 hours. The next step will only begin after this incubation. This incubation time variation depends on the type of microorganisms that may be present in the sample to be analyzed, that is, in case there are present microorganisms of the rapid growth type, the incubation time will necessarily be less than the incubation time of the fastidious growth type microorganisms.

In a more preferred embodiment, to the concentrated volume obtained in step i) it is added a volume of concentrated liquid culture medium, chosen among CLED, Ektoen, XLD, MacConkey, Bengal Rose, Cetrimide or combinations thereof, among others known by those skilled in the art and which are applicable according to the sample being analyzed. The ratio between the volume of the culture medium and the volume of the concentrated sample is 1:1 and is in the range of 200 µL to 2 mL.

In another preferred embodiment, markers (biochemicals, chemiluminescents markers), such as HMGB 1, RAGE or others known by those skilled in the art, are optionally added in sample incubation step ii), to bind to the microbial cell walls or enter their cytoplasm, thus increasing the signal emitted by each cell, directly or by means of physical, chemical or biochemical reaction, to be detected in the subsequent steps.

iii) Introduction of the sample, concentrated and enriched with the culture medium, obtained in step ii), in a reservoir matrix comprising at least two wells and at least two controlled release reservoirs of antimicrobial agents.

Each well of the reservoir matrix is configured to receive a portion of the sample (which can be the same sample - in the case of testing different antimicrobials, or different samples, in a multiple sample analysis) and is associated with a controlled release reservoir of antimicrobial agents.

Each controlled release reservoir of antimicrobial agents is configured to release an antimicrobial agent over a portion of the sample in the corresponding well by means of an injection nozzle.

iv) First analysis of the sample, concentrated and enriched with the culture medium, obtained in step ii), by irradiating it with external energy and subsequent digital recording of the information from the first analysis.

It is important to mention that the purpose of irradiating the sample in this step is to create conditions that allow the detection of the presence of microorganisms and their activity in the sample under a rapid analysis.

When determining micromovement and subsequent detection of microorganisms, this micromovement being an indication of response to the antimicrobial challenge, the process of determination of the antimicrobial susceptibility profile thereof will proceed according to the below described steps.

As indicated, the external energy is originated from coherent light sources having wavelengths varying in the range from 193 nm to 2940 nm, preferably 690 to 1064 nm, and powers varying in a range from 0,1 mW to 400 mW, preferably from 0,5 mW to 100 mW.

v) Controlled release of a first amount of at least one antimicrobial agent - which is defined by reason of the type of sample to be analyzed - from a controlled release reservoir of antimicrobial agents, over a portion of a sample disposed in the well associated with the respective controlled release reservoir of antimicrobial agents.

In one preferred embodiment, the introduction of the concentrated and enriched sample and of a first amount of antimicrobials is carried out with different amounts or concentrations both of the sample as of the antimicrobials, allowing in this manner the simultaneous processing and in a controlled manner the exposure thereof to the external energy to be used in the next step. It is possible, thus, for the sample to be processed simultaneously applying several wavelengths and different amounts or concentrations of antimicrobials, leading to a more rapid and assertive analysis of the antimicrobial susceptibility of the sample. In a preferred embodiment, the first amount of antimicrobials ranges from 1 µg to 10 mg and the concentrations range from 1 µg/L to 10 mg/L.

In yet another preferred embodiment, the introduction of different antimicrobials and different amounts or concentrations, occurs in a gradual (controlled release) and individual manner in each one of the at least two reservoirs, being equally executed a digital recording for each condition tested.

vi) Second analysis of the sample obtained in step v), which is again irradiated with external energy and subsequent digital recording of information from the second analysis for a short time period (seconds) allowing the evaluation of the activity conditions of the sample.

In a preferred embodiment, the digital recording will be carried out by means of video or image, for each condition tested.

In a preferred embodiment, the digital recordings obtained in each step undergo a digital information processing procedure resorting to the analysis of texture and of patterns, which will allow on one side evaluating and characterizing the microbial motility, and on the other side, characterize the type of micromovement that may exist in the same, being eventually possible to identify the microorganism being analyzed.

vii) Processing of the information obtained in steps iv) and vi) using a suitable processing algorithm, which is carried out preferably placing the information in a scheme having rapid and easy interpretation (meeting the great number of variables and physical parameters that may come to be tested simultaneously and throughout the process) suitable to the several purposes to which this procedure intends to provide response, being issued for the final user the intended antimicrobial susceptibility profile for each initial sample of step v).

Steps v) and vi) are repeated at least once, whereby in a repetition of step v) there is the controlled release of a second amount of at least one antimicrobial agent from a controlled release reservoir of antimicrobial agents over the respective associated well including the portion of the sample to be analyzed by means of an injection nozzle and, in a repetition of step vi), the digital recording of the corresponding analysis information is performed.

In a preferred embodiment, the added amount of at least one antimicrobial agent in a repetition of step v) is greater than, less than or equal to the amount added in the previous step v), wherein the amounts range from 1 µg to 10 mg and the concentrations range from 1 µg/L to 10 mg/L.

The result of the intended antimicrobial susceptibility profile for each initial sample is presented in the form of "susceptible/resistant" binary data or with an indication of the minimum inhibitory concentration for each antimicrobial tested.

It is also the object of this invention a viral detection process in clinical, environmental or food samples, which comprises the following steps:
i) Concentration of the microbiological component of a sample to be analyzed

As well as in the detection process carried out for bacteria and fungi, the concentration of the sample is carried out by means of several techniques that are known in the art, on their own or in progressive steps, depending on the type of sample, such as: adsorption (by ionic change in resin or metallic hydroxides), partition (in two-phase aqueous phase), centrifugation (simple, differential or density gradient), filtration (simple, electropositive or negative), selective immobilization, separation by biochemical affinity or immune magnetic (lectin, antibodies, bacteriophages or their ligands), among others, all in a macro or micro volumetric scale (liter to microliter). The technique to be used will depend on the sample to be purified, whereby the referred techniques are well known to a person skilled in the art.

In one aspect of the invention, the final concentration will have volumes that vary between the milli or microliters, preferably, the volume of the concentrated sample is in the range of 200 µL to 2 mL. Following, upon conclusion of the concentration step, the temperature of the concentrated sample is stabilized in the range from 22 °C to 45 °C according to the type of sample to be analyzed.

ii) Transfer of the sample, obtained in step i), to a cell line;
In this step ii), the concentrated sample to be analyzed is transferred to a cell line. In a preferred embodiment of the invention, the cell line is a cell line for viral growth known from the state of the art and depends on the viral type to be tested, being selected from the group consisting of Huh-7 and BHK21 cells, but not limited to them.

In another preferred embodiment, markers (biochemicals, chemiluminescents markers), such as HMGB 1, RAGE or others known by those skilled in the art, are optionally added in sample incubation step ii), to bind to the microbial cell walls or enter their cytoplasm, thus increasing the signal emitted by each cell, directly or by means of physical, chemical or biochemical reaction, to be detected in the subsequent steps.

The transfer to the cell line is carried out by inoculation and then the cell lines growth variation by viral action is verified.

iii) Analysis of the sample obtained in step ii), which is irradiated by means of the external energy and subsequent execution of the digital recording of the information from the analysis
As in the detection process carried out for bacteria and fungi, the purpose of irradiating the sample is to create conditions that allow for the rapid detection of the presence of viruses.

As indicated, the external energy is originated from coherent light sources having wavelengths varying in the range from 193 nm to 2940 nm, preferably 690 to 1064 nm, and powers varying in a range from 0,1 mW to 400 mW, preferably from 0,5 mW to 100 mW.

iv) Processing of the information obtained in step iii) using a suitable processing algorithm, with the result being issued to the final user in the form of detected / not detected binary data for each sample of step i)
The processing of the information obtained in step iii) is preferably carried out using a suitable processing algorithm, by placing the information in an easy and quick interpretation scheme (given the large number of variables and physical parameters that may be tested simultaneously and throughout the process) suitable for the various purposes to which this procedure is intended to respond.

In case of detection of virus, the process being considered will be applied up to step iii). The interpretation of the information is next obtained and the result is presented in the form of "detected/not detected" binary data.

### EXAMPLES

The examples described below have only the purpose of illustrating the present invention and are not intended to limit the same.

### Example 1

A sample of 5 ml of blood was submitted to an antimicrobial susceptibility analysis for *Pseudomonas aeruginosa.*

In a first step the purification of the sample was carried out, by means of the eukaryotic cell lysis of the sample and subsequent concentration by centrifugation, with collection of supernatants.

Next, 0,5 ml to 1 ml of the concentrated sample was enriched with the addition of 0,5 ml to 1 ml of doubly concentrated Cetrimide culture medium. The enriched sample was incubated at a temperature of 37 °C, for an interval from 5 to 60 minutes, until the micromovements appear.

After the concentration and incubation, the sample was introduced in a matrix of 8 wells, each one coupled to an antimicrobial reservoir comprising ceftazidime, cefepime, piperacillin+tazobactam, ciprofloxacin, amikacin, imipenem, aztreonam, amoxicillin-clavulanic acid, to release them in the wells in order to obtain concentrations that vary from 1 to 100 ug/ml.

A first analysis of the sample was carried out, by shining coherent light (laser having 650 nm wavelength, 10mW power) simultaneously on the different elements of the matrix with collection of the digital information by reflection of the energy emitted, with a frequency of 4 digital readings per second, with a duration of 5 seconds each reading.

Last, the processing was carried out, that is, the texture of the videos or sequence of images obtained was analyzed and the conversion of the result obtained in information to the final user: susceptible/resistant and/or MIC.

It was verified that for the antimicrobials ceftazidime, cefepime, imipenem, and aztreonam no micromovement was detected in the sample in the concentration intervals which vary from 1 to 100 ug/ml. For the antibiotics piperacillin+tazobactam, ciprofloxacin, amikacin and amoxicillin-clavulanic acid micromovement was detected whereby the final result presented was the minimum inhibitory concentration for each one of them, the CIM refers to the lower concentration of antimicrobial wherein the bacteria is not able to grow (present micromovement), varying between 1 and 100 ug/ml.

### Example 2

A soil sample was submitted to an antimicrobial susceptibility analysis for *Mucor indicus.*

In a first step, the sample was purified by diluting a sample of 5 g of soil in 100 ml of saline solution, followed by filtration in a sequence of sterile filters, from 100 to 1 micrometer of pore for each filter, the sample was then concentrated by centrifugation and followed by collection of the supernatant.

Next, 1 ml of the concentrated sample was enriched, adding 1 ml double concentrated *Bengal Rose* culture medium. This sample was submitted to incubation for 40 minutes, at a temperature of 25 °C.

After the concentration and incubation, the sample was distributed in a matrix having 4 wells and 4 reservoirs, each one with fluconazole, amphotericin b, metronidazole, clotrimazole, wherein the concentrations of these antimicrobials vary from 1 to 100 ug/ml.

The analysis of the sample was carried out by shining coherent light (laser with 650 nm wavelength, 10mW power) simultaneously on the different elements of the matrix with collection of the digital information by reflection of the energy emitted, with a frequency of 4 digital readings per second, with a duration of 5 seconds each reading.

Last, the interpretation was carried out, that is, the texture of the video or sequence of images obtained was analyzed and the conversion of the result obtained in information to the final user: susceptible/resistant and/or MIC.

For the anti-fungic fluconazole, amphotericin b, metronidazole, clotrimazole, micromovement was detected, whereby the final result presented was the minimum inhibitory concentration for each one of them, that is, the CIM refers to lower concentration of anti-fungi where the fungi is not able to grow (present micromovement), varying from 1 to 100 ug/ml.

### Example 3

A sample of milk was submitted to viral detection analysis, specifically the presence of the Norwalk virus was analyzed.

In a first step, 5 ml of the sample was submitted to eukaryotic cell lysis by sonication, and subsequent adsorption of viral particles, followed by centrifugation and collection of the supernatant.

Next, 1 ml of the purified sample was added to a cell line (for example: Huh-7, BHK21 or other, known from the state of the art) and subsequently incubated for 60 minutes, at a temperature of 37 °C.

After the concentration and transfer to a cell line, the enriched sample was distributed in a matrix of 4 wells, with chemiluminescent biomarkers (for example HMGB 1, RAGE and others), with concentrations of these biomarkers varying from 1 to 10 ug/ml.

The analysis of the sample was carried out by shining coherent light (laser with 650 nm wavelength, 10mW power) simultaneously on the different elements of the matrix with collection of the digital information by reflection of the energy emitted, with a frequency of 4 digital readings per second, with a duration of 5 seconds each reading.

Last, the interpretation was carried out, that is, the texture of video or the sequence of images obtained was analyzed and the conversion of the result obtained in information to the final user: detected/not detected.

### LIST OF CITATIONS

Follows the list of citations:

### PATENT LITERATURE

- WO2017198240A1
- JP2019088339A

### NON-PATENTARY LITERATURE:

- Han, S., No, H., Baek, Y., Park, H., Lee, K., Yang, S., & Park, Y. (2019). Rapid antimicrobial susceptibility test using spatiotemporal analysis of laser speckle dynamics of bacterial colonies. bioRxiv.

## Claims

1. Process for microbiological detection and determination of antimicrobial susceptibility in clinical, environmental or food samples **characterized by** comprising the following steps:
i) Concentration of the microbiological component of a sample to be analyzed;
ii) Incubation of the sample, obtained in step i), and addition of a volume of a concentrated liquid culture medium; wherein the ratio between the volume of the culture medium and the volume of the concentrated sample is of 1: 1 and is in the range of 200 µL to 2 mL;
iii) Introduction of the concentrated and enriched sample, obtained in step ii), in a reservoir matrix comprising at least two wells and at least two controlled release reservoirs of antimicrobial agents, wherein each well is configured to receive a portion of the sample and is associated to a controlled release reservoir of antimicrobial agents, wherein each controlled release reservoir of antimicrobial agents is configured to release an antimicrobial agent over a portion of the sample;
iv) First analysis of the sample enriched with the culture medium, obtained in step ii), by irradiating it with external energy and subsequent digital recording of the information from the first analysis to detect the presence of micromovements, wherein the external energy is originated from coherent light sources having wavelengths varying in the range from 193 nm to 2940 nm, and powers varying in a range from 0,5 mW to 400 mW;
v) Controlled release of a first amount of at least one antimicrobial agent, from a controlled release reservoir of antimicrobial agents, over a portion of the sample obtained in step ii), which is disposed in the well associated with the respective controlled release reservoir of antimicrobial agents;
vi) Second analysis of the sample obtained in step v), which is again irradiated with external energy and subsequent digital recording of information from the second analysis;
vii) Processing of the information obtained in steps iv) and vi) using a suitable processing algorithm, with the antimicrobial susceptibility profile being issued to the end user as a result of each sample from step v);
wherein steps v) and vi) are repeated at least once, wherein in a repetition of step v) the controlled release of a second amount of at least one antimicrobial agent from a controlled release reservoir of antimicrobial agents occurs, over the respective associated well including the sample portion and in which in a repetition of step vi) the digital recording of the corresponding analysis information is performed;
wherein the coherent light optical properties and the reflection and / or refraction thereof allows the detection and characterization of the micromovements of the microorganisms; and
wherein the microorganisms to be detected are selected from the group which consists of bacteria or fungi having rapid growth or fastidious growth.

2. Process, according to claim 1, **characterized by** upon conclusion of step i) the temperature of the sample to be examined being stabilized in the range from 22 °C to 45 °C.

3. Process, according to any one of the preceding claims, **characterized by** the concentrated sample of step ii) remaining in incubation for a period less than or equal to 12 hours.

4. Process, according to any one of the preceding claims, **characterized by** the concentrated liquid culture medium added in step ii) being chosen among CLED, Ektoen, XLD, MacConkey, Bengal Rose and Cetrimide.

5. Process, according to any one of the preceding claims, **characterized by** the addition of signal enhancing markers emitted by microbial cells chosen among biochemicals, chemiluminescents markers in step iii).

6. Process, according to claim 5, **characterized by** the chemiluminescent markers being chosen among HMGB 1 and RAGE.

7. Process, according to claim 1, **characterized by** the coherent light sources being chosen among argon fluoride, the Helium-Neon, YAG or carbon dioxide or a combination thereof.

8. Process, according to any one of the preceding claims, **characterized by** in step v), the controlled release of the antimicrobial agent being accomplished by adding different amounts or concentrations of antimicrobial over the portion of concentrated sample disposed in the associated well by means of an injection nozzle, wherein the added amount of at least one antimicrobial agent in a repetition of step v) is greater than, less than or equal to the amount added in the previous step v), wherein the amounts range from 1 µg to 10 mg and the concentrations range from 1 µg/L to 1 mg/L.

9. Process, according to any one of the preceding claims, **characterized by** the digital recording of steps iv) and vi) being carried out by means of video or image, for each condition tested.

10. Process, according to any one of the preceding claims, **characterized by** the digital recordings of steps iv) and vi) comprising the processing of the digital information resorting to the analysis of texture and of patterns.

11. Process, according to any one of the preceding claims, **characterized by** in step vii) being issued to the final user the result of the antimicrobial susceptibility profile for each initial sample, in the form of a susceptible / resistant binary data or having an indication of the minimum inhibitory concentration for each antimicrobial tested.

## Patentansprüche

1. Verfahren zum mikrobiologischen Nachweis und zur Bestimmung antimikrobieller Empfindlichkeit in klinischen, Umwelt- oder Lebensmittelproben, **dadurch gekennzeichnet, dass** es die folgenden Schritte umfasst:
i) Konzentrierung der mikrobiologischen Komponente einer zu analysierenden Probe;
ii) Inkubation der in Schritt i) gewonnenen Probe und Zugabe eines Volumens eines konzentrierten flüssigen Nährmediums, wobei das Volumenverhältnis zwischen dem Nährmedium und der konzentrierter Probe 1:1 beträgt und die Menge im Bereich von 200 µl bis 2 ml liegt;
iii) Einbringung der in Schritt ii) gewonnenen konzentrierten und angereicherten Probe in eine Reservoirmatrix, die mindestens zwei Vertiefungen und mindestens zwei Reservoirs zur kontrollierten Freisetzung antimikrobieller Wirkstoffe aufweist, wobei jede Vertiefung so ausgelegt ist, dass sie einen Teil der Probe aufnimmt und einem Reservoir zur kontrollierten Freisetzung antimikrobieller Wirkstoffe zugeordnet ist, wobei jedes Reservoir zur kontrollierten Freisetzung antimikrobieller Wirkstoffe so ausgelegt ist, dass es einen antimikrobiellen Wirkstoff über einen Teil der Probe freisetzt;
iv) erste Analyse der mit dem Nährmedium angereicherten, in Schritt ii) gewonnenen Probe durch Bestrahlung mit externer Energie sowie eine sich daran anschließende digitale Aufzeichnung der bei der ersten Analyse gewonnenen Informationen, um das Vorhandensein von Mikrobewegungen festzustellen, wobei die externe Energie von kohärenten Lichtquellen mit Wellenlängen im Bereich von 193 nm bis 2940 nm und Leistungen im Bereich von 0,5 mW bis 400 mW stammt;
v) kontrollierte Freisetzung einer ersten Menge mindestens eines antimikrobiellen Wirkstoffs aus einem Reservoir mit kontrollierter Freisetzung antimikrobieller Wirkstoffe über einen Teil der in Schritt ii) gewonnenen Probe, die in der Vertiefung angeordnet ist, die dem jeweiligen Reservoir mit kontrollierter Freisetzung antimikrobieller Wirkstoffe zugeordnet ist;
vi) zweite Analyse der in Schritt v) gewonnenen Probe, die erneut mit externer Energie bestrahlt wird, und sich daran anschließende digitale Aufzeichnung der bei dieser zweiten Analyse gewonnenen Informationen;
vii) Weiterverarbeitung der in den Schritten iv) und vi) gewonnenen Informationen unter Verwendung eines geeigneten Verarbeitungsalgorithmus, wobei dem Endbenutzer als Ergebnis jeder Probe aus Schritt v) das antimikrobielle Empfindlichkeitsprofil ausgegeben wird;
wobei die Schritte v) und vi) mindestens einmal wiederholt werden, wobei bei einer Wiederholung von Schritt v) die kontrollierte Freisetzung einer zweiten Menge mindestens eines antimikrobiellen Wirkstoffs aus einem Reservoir mit kontrollierter Freisetzung antimikrobieller Wirkstoffe über die jeweils zugeordneten Vertiefung, die den Teil der Probe enthält, erfolgt und wobei bei einer Wiederholung von Schritt vi) die digitale Aufzeichnung der entsprechenden Analyseinformationen erfolgt;
wobei die optischen Eigenschaften des kohärenten Lichts und dessen Reflexion bzw. Brechung den Nachweis und Charakterisierung der Mikrobewegungen der Mikroorganismen ermöglichen; und
wobei die nachzuweisenden Mikroorganismen aus der Gruppe ausgewählt sind, die aus schnell wachsenden oder anspruchsvollen Bakterien oder Pilzen besteht.

2. Verfahren nach Anspruch 1, **dadurch gekennzeichnet, dass** nach Abschluss von Schritt i) die Temperatur der zu analysierenden Probe im Bereich von 22 °C bis 45 °C stabilisiert wird.

3. Verfahren nach einem der vorhergehenden Ansprüche, **dadurch gekennzeichnet, dass** die konzentrierte Probe aus Schritt ii) für einen Zeitraum kleiner als oder gleich 12 Stunden in Inkubation verbleibt.

4. Verfahren nach einem der vorhergehenden Ansprüche, **dadurch gekennzeichnet, dass** das in Schritt ii) zugegebene konzentrierte flüssige Nährmedium aus CLED, Ektoen, XLD, MacConkey, Bengal Rose und Cetrimid ausgewählt ist.

5. Verfahren nach einem der vorhergehenden Ansprüche, **dadurch gekennzeichnet, dass** von mikrobiellen Zellen emittierten signalverstärkenden Markern, die aus biochemischen und chemilumineszierenden Markern in Schritt iii) zugegeben werden.

6. Verfahren nach Anspruch 5, **dadurch gekennzeichnet, dass** die chemilumineszierende Marker aus HMGB1 und RAGE ausgewählt sind.

7. Verfahren nach Anspruch 1, **dadurch gekennzeichnet, dass** die kohärenten Lichtquellen aus Argonfluorid, Helium-Neon, YAG oder Kohlendioxid oder einer Kombination davon ausgewählt sind.

8. Verfahren nach einem der vorhergehenden Ansprüche, **dadurch gekennzeichnet, dass** in Schritt v) die kontrollierte Freisetzung des antimikrobiellen Wirkstoffs durch Zugabe unterschiedlicher Mengen oder Konzentrationen des antimikrobiellen Wirkstoffs über den Teil der konzentrierten Probe, die in der zugeordneten Vertiefung angeordnet ist, mittels einer Injektionsdüse erfolgt, wobei die zugegebene Menge des mindestens einen antimikrobiellen Wirkstoffs bei einer Wiederholung von Schritt v) größer, kleiner oder gleich der im vorhergehenden Schritt v) zugegebenen Menge ist, wobei die Mengen im Bereich von 1 µg bis 10 mg und die Konzentrationen im Bereich von 1 µg/l bis 1 mg/l liegen.

9. Verfahren nach einem der vorhergehenden Ansprüche, **dadurch gekennzeichnet, dass** die digitale Aufzeichnung der Schritte iv) und vi) mittels Video oder Bild für jede getestete Bedingung erfolgt.

10. Verfahren nach einem der vorhergehenden Ansprüche, **dadurch gekennzeichnet, dass** die digitalen Aufzeichnungen der Schritte iv) und vi) die Verarbeitung der digitalen Informationen unter Rückgriff auf die Analyse von Textur und Mustern umfassen.

11. Verfahren nach einem der vorhergehenden Ansprüche, **dadurch gekennzeichnet, dass** in Schritt vii) dem Endbenutzer das Ergebnis des antimikrobiellen Empfindlichkeitsprofils für jede Ausgangsprobe in Form von binären Empfindlichkeits- bzw. Resistenzdaten oder mit einer Angabe der minimalen Hemmkonzentration für jeden getesteten antimikrobiellen Wirkstoff ausgegeben wird.

## Revendications

1. Procédé de détection microbiologique et de détermination de la sensibilité aux antimicrobiens dans des échantillons cliniques, environnementaux ou alimentaires **caractérisé en ce qu'**il comprend les étapes suivantes :
i) Concentration du composant microbiologique d'un échantillon à analyser ;
ii) Incubation de l'échantillon, obtenu à l'étape i), et ajout d'un volume d'un milieu de culture liquide concentré ; dans lequel le rapport entre le volume du milieu de culture et le volume de l'échantillon concentré est de 1:1 et est dans la plage de 200 µL à 2 mL ;
iii) Introduction de l'échantillon concentré et enrichi, obtenu à l'étape ii), dans une matrice réservoir comprenant au moins deux puits et au moins deux réservoirs à libération contrôlée d'agents antimicrobiens, dans laquelle chaque puits est configuré pour recevoir une partie de l'échantillon et est associé à un réservoir à libération contrôlée d'agents antimicrobiens, dans lequel chaque réservoir à libération contrôlée d'agents antimicrobiens est configuré pour libérer un agent antimicrobien sur une partie de l'échantillon ;
iv) Première analyse de l'échantillon enrichi avec le milieu de culture, obtenu à l'étape ii), en l'irradiant avec une énergie externe et l'enregistrement numérique ultérieur des informations provenant de la première analyse pour détecter la présence de micro-mouvements, dans lequel l'énergie externe provient de sources de lumière cohérente ayant des longueurs d'onde variant dans la plage de 193 nm à 2940 nm et des puissances variant dans une plage de 0,5 mW à 400 mW ;
v) Libération contrôlée d'une première quantité d'au moins un agent antimicrobien, provenant d'un réservoir à libération contrôlée d'agents antimicrobiens, sur une partie de l'échantillon obtenu à l'étape ii), qui est disposé dans le puits associé au réservoir à libération contrôlée respectif de agents antimicrobiens ;
vi) Seconde analyse de l'échantillon obtenu à l'étape v), qui est à nouveau irradié avec de l'énergie externe et l'enregistrement numérique ultérieur des informations provenant de la deuxième analyse ;
vii) Traitement des informations obtenues aux étapes iv) et vi) à l'aide d'un algorithme de traitement approprié, avec le profil de sensibilité aux antimicrobiens étant délivré à l'utilisateur final en tant que résultat de chaque échantillon de l'étape v) ;
dans lequel les étapes v) et vi) sont répétées au moins une fois, dans lequel, lors d'une répétition de l'étape v), la libération contrôlée d'une seconde quantité d'au moins un agent antimicrobien provenant d'un réservoir à libération contrôlée d'agents antimicrobiens se produit, sur le puits associé respectif comprenant la partie d'échantillon et dans laquelle, lors d'une répétition de l'étape vi), l'enregistrement numérique des informations d'analyse correspondantes est effectué ;
dans lequel les propriétés optiques de la lumière cohérente et la réflexion et / ou réfraction de celle-ci permet la détection et la caractérisation des micro-mouvements des micro-organismes ; et
dans lequel les micro-organismes à détecter sont choisis parmi le groupe constitué de bactéries ou de champignons ayant une croissance rapide ou une croissance fastidieuse.

2. Procédé selon la revendication 1, **caractérisé en ce qu'**à la fin de l'étape i), la température de l'échantillon à examiner est stabilisée dans la plage de 22 °C à 45 °C.

3. Procédé, selon l'une quelconque des revendications précédentes, **caractérisé en ce que** l'échantillon concentré de l'étape ii) reste en incubation pendant une période inférieure ou égale à 12 heures.

4. Procédé selon l'une quelconque des revendications précédentes, **caractérisé en ce que** le milieu de culture liquide concentré ajouté à l'étape ii) est choisi parmi CLED, Ektoen, XLD, MacConkey, Bengal Rose et Cetrimide.

5. Procédé, selon l'une quelconque des revendications précédentes, **caractérisé par** l'ajout de marqueurs d'amélioration de signal émis par les cellules microbiennes choisis parmi les marqueurs biochimiques, chimioluminescents à l'étape iii).

6. Procédé selon la revendication 5, **caractérisé en ce que** les marqueurs chimioluminescents sont choisis parmi HMGB1 et RAGE.

7. Procédé selon la revendication 1, **caractérisé en ce que** les sources de lumière cohérente sont choisies parmi le fluorure d'argon, l'Hélium-Néon, le YAG ou le dioxyde de carbone ou une combinaison de ceux-ci.

8. Procédé selon l'une quelconque des revendications précédentes, **caractérisé en ce qu'**à l'étape v), la libération contrôlée de l'agent antimicrobien est réalisée par l'ajout de différentes quantités ou concentrations d'antimicrobien sur la partie d'échantillon concentré disposée dans le puits associé au moyen d'une buse d'injection, dans laquelle la quantité ajoutée d'au moins un agent antimicrobien lors d'une répétition de l'étape v) est supérieure, inférieure ou égale à la quantité ajoutée lors de l'étape précédente v), dans laquelle les quantités vont de 1 µg à 10 mg et les concentrations varient de 1 µg/L à 1 mg/L.

9. Procédé, selon l'une quelconque des revendications précédentes, **caractérisé en ce que** l'enregistrement numérique des étapes iv) et vi) est effectué au moyen d'une vidéo ou d'une image, pour chaque condition testée.

10. Procédé selon l'une quelconque des revendications précédentes, **caractérisé par** les enregistrements numériques des étapes iv) et vi) comprenant le traitement des informations numériques faisant appel à l'analyse de texture et de motifs.

11. Procédé, selon l'une quelconque des revendications précédentes, **caractérisé en ce qu'**à l'étape vii) est délivré à l'utilisateur final le résultat du profil de sensibilité antimicrobienne pour chaque échantillon initial, sous la forme d'une donnée binaire sensible / résistante ou ayant une indication de la concentration minimale inhibitrice pour chaque antimicrobien testé.
